(19) Europäisches Patentamt
European Patent Office
Office européen des brevets

(11) EP 0 676 393 B1

(12) **FASCICULE DE BREVET EUROPEEN**

(45) Date de publication et mention de la délivrance du brevet: **19.08.1998 Bulletin 1998/34**

(21) Numéro de dépôt: **95103729.0**

(22) Date de dépôt: **15.03.1995**

(51) Int Cl.[6]: **C07C 403/14**, C07C 403/08, C07C 49/303, C07C 49/543, C07C 49/557, C07C 31/135, C07C 33/14, C07C 45/30, A61K 7/46, C11B 9/00

(54) **Cétones cycliques nouvelles et leur utilisation en parfumerie**

Cyclische Ketone und deren Verwendung als Riechstoffe

Cyclic ketones and their use in perfumery

(84) Etats contractants désignés:
**CH DE FR GB LI NL**

(30) Priorité: **08.04.1994 CH 1047/94**

(43) Date de publication de la demande:
**11.10.1995 Bulletin 1995/41**

(73) Titulaire: **FIRMENICH SA**
**1211 Genève 8 (CH)**

(72) Inventeurs:
- **Schulte-Elte, Karl-Heinrich**
  **CH-1213 Onex (CH)**
- **Chapuis, Christian**
  **CH-1295 Mies (CH)**
- **Pamingle, Hervé**
  **CH-1290 Versoix (CH)**
- **Blanc, Pierre-Alain**
  **CH-1263 Crassier (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**EP-A- 0 449 034**
**CH-A- 513 096**
**CH-A- 553 187**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 676 393 B1

Printed by Jouve, 75001 PARIS (FR)

## Description

La présente invention a trait à des composés nouveaux utiles dans la parfumerie. Elle concerne, plus particulièrement, des cétones cycliques de formule

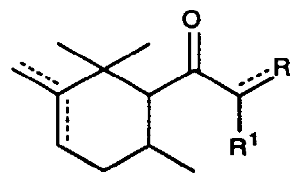

(I)

dans laquelle les lignes pointillées indiquent l'emplacement d'une liaison simple ou double, le symbole $R^1$ représente un atome d'hydrogène ou un radical méthyle et le symbole R représente l'hydrogène ou un radical alkyle, alcényle ou alkylidène inférieur, linéaire ou ramifié.

Par radical inférieur, on entend ici un radical contenant de 1 à 4 atomes de carbone. Des exemples de groupes R incluent les radicaux méthyle, éthyle, propyle, butyle, isopropyle, méthylène, vinyle, 1-propényle, allyle, éthylidène, isopropylidène et propylidène.

Les cétones (I) sont des composés de structure nouvelle et possèdent des propriétés odorantes originales. Ceci est d'autant plus surprenant que l'on connaît déjà bon nombre de composés odorants dont la structure est proche de celle des cétones (I), de sorte que l'on aurait pu s'attendre à ce qu'il soit impossible, ou du moins fort improbable, que des composés nouveaux de structure analogue puissent encore apporter une contribution olfactive originale et avantageuse à la palette du parfumeur.

Or, c'est cette abondance même de composés odorants de structure analogue dont le parfumeur dispose actuellement qui explique que la structure et les propriétés odorantes des cétones (I) soient restées inconnues jusqu'à ce jour et qui confère un caractère inattendu à la présente invention.

Car nous venons maintenant de constater que les cétones (I), dont la structure est proche de celle des damascones et damascénones tant appréciées en parfumerie, possèdent toutefois des odeurs distinctes de, et même plus variées que, celles de ces dernières, pouvant se prêter ainsi à un usage plus étendu et en tout cas tout à fait original par rapport à celui des composés connus.

Le brevet CH-B-513 096 décrit la préparation de tels composés, qui diffèrent des cétones de la présente invention principalement par la position de la double laison endocyclique, qui se trouve entre les carbones 1 et 2 ou 2 et 3 et, dans certains cas, par des substituants du cycle cyclohéxyle, en particulier la présence d'un atome d'hydrogène en position 6.

Ces cétones nouvelles développent, en effet, des notes odorantes où les caractères fleuri, fruité typiques des damascones se trouvent combinés à des notes aromatiques poudrées, balsamiques, myrrhe et iris plus proches des odeurs typiques des irones et composés analogues. C'est justement cette combinaison de caractères odorants qui rend les cétones (I) particulièrement utiles en parfumerie, car elles permettent à elles seules d'impartir à des compositions parfumantes toute une gamme de nuances olfactives dont on n'aurait pu disposer, en principe, qu'avec la contribution de plusieurs des composés connus cités et dont la combinaison produirait cependant des effets olfactifs inévitablement différents.

Ainsi, la présente invention apporte une contribution nouvelle et inattendue au problème permanent de la recherche de nouveaux ingrédients parfumants, susceptibles d'une synthèse industrielle et d'un emploi divers et au moins tout aussi avantageux que celui des composés connus et disponibles sur le marché. En plus, elle est l'exemple type de l'imprévisibilité des qualités olfactives de composés nouveaux, même dans des domaines structurels où des recherches antérieures ont été richement productives et où l'homme de l'art ne trouverait aucune motivation pour continuer à approfondir.

Les odeurs des composés de l'invention sont, par ailleurs, fort puissantes et substantives et se prêtent à une gamme très variée d'applications. On peut en effet les employer dans la préparation de compositions et bases parfumantes, parfums et eaux de toilette, tant comme dans le parfumage d'articles de consommation divers, tels que les savons, les gels de douche ou bain, les shampoings et autres produits d'hygiène capillaire, les préparations cosmétiques et les désodorisants corporels ou d'air ambiant. Ils se révèlent aussi d'un emploi avantageux pour le parfumage de détergents et adoucissants textiles, ainsi que de produits d'entretien.

Dans ces applications, les cétones (I) peuvent être utilisées seules ou, comme il est plus courant, en combinaison avec d'autres ingrédients parfumants, solvants et adjuvants d'usage habituel. Les concentrations dans lesquelles elles peuvent être employées varient dans une gamme de valeurs très étendue qui est fonction de la nature des autres ingrédients dans une composition donnée, ainsi que de l'effet olfactif que l'on cherche à obtenir et de la nature du

produit que l'on veut parfumer. On peut citer à titre d'exemple des concentrations de l'ordre de 1 à 10%, ou même 20% ou plus en poids de cétone (I), par rapport au poids de composition ou concentré parfumant dans lequel cette cétone est incorporée. Des valeurs bien inférieures à celles citées seront typiquement utilisées lorsqu'il s'agit d'employer les cétones (I) pour le parfumage des articles divers cités auparavant.

En dépit de leurs caractères olfactifs communs cités plus haut, les composés (I) possèdent tous d'autres nuances olfactives qui rendent leur odeur distincte d'un composé à l'autre et qui permettent d'obtenir des fragrances variées selon que l'on utilise l'un ou l'autre de ces composés. Par ailleurs, en raison de leur structure, ils peuvent se présenter sous différentes formes isomériques, qui peuvent être optiquement actives ou racémiques, chacun des isomères pouvant présenter des qualités odorantes distinctes.

Parmi les composés qui représentent au mieux l'invention, on peut citer à titre préférentiel la (E)-1-(2,2,c-3,t-6-tétraméthyl-r-1-cyclohexyl)-2-pentén-1-one qui développe une odeur fleurie, fruitée qui rappelle celle des damascones et aussi un caractère ironé, myrrhe résinoïde, olibanum et violette.

Un autre composé préféré de l'invention et dont le caractère fruitédamasconé est plus puissant et élégant, est la cis-(E)-1-(2,2,6-triméthyl-3-méthylène-1-cyclohexyl)-2-butén-1-one. La 1-(2,2,c-3,t-6-tétraméthyl-r-1-cyclohexyl)-1-propanone possède elle une odeur très puissante, où les caractères boisé, poudreux, myrrhe sont dominants, alors que la cis-1-(2,2,3,6-tétraméthyl-3-cyclohexén-1-yl)-1-propanone développe une note de tête fruitée-damascone, gentiane, accompagnée de caractères de fond de type aromatique, camphré, herbacé et fenchylique.

Les odeurs d'autres composés selon l'invention sont présentées plus loin.

D'une façon générale, nous avons constaté que parmi les isomères possibles des composés de formule (I) possédant un cycle saturé, ceux dont la structure est représentée par la formule

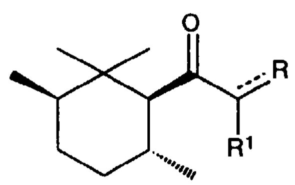

(Ia)

dans laquelle la ligne pointillée et les symboles R et $R^1$ ont le sens indiqué à la formule (I), et les trois substituants ont les positions relatives indiquées, présentaient au mieux les caractères olfactifs recherchés. Les composés de formule (I) peuvent se présenter sous forme racémique ou sous forme de l'un des énantiomères optiquement actifs.

L'invention concerne également un procédé pour la préparation d'un composé de formule (I) telle que définie à la revendication 1, caractérisé en ce qu'il comprend la réaction, avec un agent oxydant, d'un alcool de formule

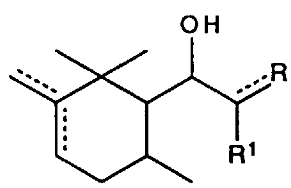

(II)

dans laquelle les lignes pointillées et les symboles $R^1$ et R ont le sens indiqué à la formule (I) et, si nécessaire, l'isomérisation de la cétone ainsi obtenue.

Les alcools de formule (II) sont des composés odorants nouveaux, qui font également l'objet de l'invention. La réaction d'oxydation de ces alcools s'effectue à l'aide d'agents oxydants d'usage courant dans ce type de réactions. Les conditions de réaction sont décrites en détail dans les exemples présentés plus loin.

Les alcools de départ peuvent être préparés à partir d'aldéhydes connus à l'aide d'une réaction dite de Grignard, selon le schéma que voici :

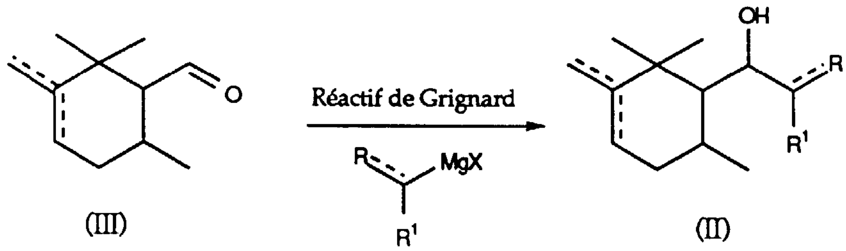


(III) (II)

Dans ce schéma, les lignes pointillées et les symboles R et $R^1$ sont définis comme à la formule (I) et le symbole X représente un atome d'halogène. Les conditions de ces réactions de Grignard sont décrites en détail plus loin.

Bien entendu, lorsque l'aldéhyde de départ est utilisé sous forme d'un isomère racémique ou optiquement actif, on obtient l'alcool (II) et la cétone (I) sous forme racémique ou sous forme de l'isomère ayant la même configuration que le produit de départ utilisé. Par exemple, on peut préparer des composés préférés de l'invention selon le schéma que voici :

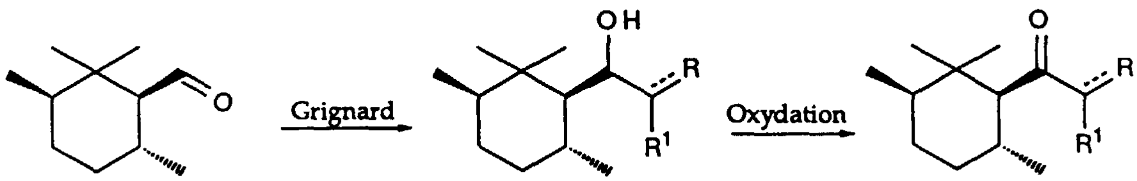


(IIa) (Ia)

L'aldéhyde dans ce schéma peut être racémique ou l'un des énantiomères ayant la configuration relative des substituants indiquée et les symboles R et $R^1$ sont définis plus haut.

La préparation des aldéhydes de formule (III) et de leurs isomères est décrite dans EP-B1-374 509 ou EP-A1-449 034 et les informations contenues dans ces publications, relatives auxdites préparations, sont inclues ici par référence.

Par ailleurs, certaines cétones (I) à chaîne latérale insaturée ont été préparées par isomérisation d'autres cétones (I) isomères appropriées qui avaient été obtenues par le procédé décrit ci-dessus.

Un exemple particulier est le suivant :

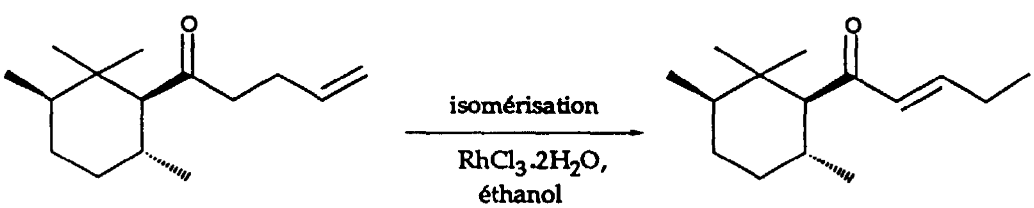


Les conditions de ces isomérisations sont décrites en détail plus loin.

L'invention sera maintenant décrite plus en détail à l'aide des exemples suivants dans lesquelles les températures sont indiquées en degrés centigrades et les abréviations ont le sens usuel dans l'art.

Exemple 1

Préparation des alcools de formule (II)

Méthode générale des réactions de Grignard

Dans un ballon sous atmosphère d'azote, equipé d'une agitation mécanique, on introduit du magnésium (x mole)

et on recouvre d'éther sulfurique ou de tétrahydrofuranne (THF) anhydre. La réaction est amorcée à l'aide d'un cristal d'iode et quelques gouttes d'halogénure d'alkyle. On introduit ensuite goutte à goutte une solution de l'halogénure approprié (x mole) dans l'éther ou le THF (la température monte à reflux). On laisse réagir à reflux jusqu'à disparition du magnésium et on ajoute alors goutte à goutte l'aldéhyde approprié (environ 0,8 x mole) dans l'éther ou le THF. La réaction est exothermique et la température monte à reflux. On laisse agiter pendant une nuit à température ambiante et on verse le mélange réactionnel sur de la glace ou de la glace et $NH_4Cl$, on extrait à l'éther, on lave jusqu'à neutralité avec $NH_4Cl$ ou de la saumure, on sèche sur $Na_2SO_4$, on concentre sous vide et on distille. Si nécessaire, l'alcool ainsi obtenu est soumis à une purification chromatographique.

On a préparé ainsi les produits suivants :

a. 1-(2,2,c-3,t-6-tétraméthyl-r-1-cyclohexyl)-4-pentén-1-ol

Préparé à partir de 2,2,c-3,t-6-tétraméthyl-r-1-cyclohexanecarbaldéhyde (8,4 g; 0,05 mole; voir EP-B1-374 509) et de 4-bromo-1-butène (8,1 g; 0,06 mole). On a obtenu le penténol désiré pur à 99%, avec un rendement ~ 45% (3 isomères c-3,t-6/t-3,c-6/t-3,t-6 63:24:12).

Les données analytiques étaient les suivantes :

P. éb. 52°/5 Pa

| | | | |
|---|---|---|---|
| IR : | 1640, 3080(- C = $CH_2$) ; 3500 (- OH) $cm^{-1}$ | | |
| RMN($^1$H,360 MHz) : | isomère majoritaire : 0,77(s,3H) ; 0,83(d,J=7Hz,3H) ; 0,88(s,3H) ; 1,03(d,J=7Hz,3H) ; 3,95 (m,1H) ; 4,98(d,J=11Hz,1H) ; 5,06 (dxd,$J_1$=1,$J_2$=18Hz,1H) ; 5,85(m,1H) δ ppm | | |
| SM : | c-3,t-6 : 224($M^+$,0) ; m/e : | 140(10), 125(53), 109(16), 95(26), 83(100), 69(54), 55 (58), 41(49) | |
| | t-3,c-6 : 224($M^+$,0) ; m/e : | 137(14), 125(58), 109(34), 95(39), 83(100), 69(62), 55 (67), 41(52) | |
| | t-3,t-6 : 224($M^+$,0) ; m/e : | 140(10), 125(60), 109(22), 95(30), 83(100), 69(57), 55 (66), 41(58) | |

b. 1-(2,2,c-3,t-6-tétraméthyl-r-1-cyclohexyl)-1-propanol

Préparé à partir de 2,2,c-3,t-6-tétraméthyl-r-1-cyclohexanecarbaldéhyde (5,6 g ; 33 mmole) et de 10,15 g de bromure d'éthyle. Le propanol désiré, pur à 92%, a été obtenu avec un rendement de 59%.

Données analytiques :

P. éb. 88°/3,5 x $10^2$ Pa

| | |
|---|---|
| IR : | 3450 $cm^{-1}$ : - OH |
| RMN($^1$H,360 MHz) : | 0,78(s,3H) ; 0,84(d,J=7Hz,3H) ; 0,88(s,3H) ; 0,95(t,J=7Hz, 3H); 1,01(d,J=7Hz,3H) ; 3,82 (dxd,$J_1$=7,$J_2$=8Hz,1H) δ ppm |
| SM : | 198($M^+$,0) ; m/e : 169(20), 151(18), 140(29), 125(91), 109(9), 95(21), 83(100), 69(65), 55 (49), 41(38) |
| Odeur : | boisée, ambrée, cuir |

c. cis-1-(2,2,3,6-tétraméthyl-3-cyclohexén-1-yl)-1-propanol

Préparé à partir de 2,2,3,6-tétraméthyl-3-cyclohexène-1-carbaldéhyde (9 mmole ; cis:trans 9:1 ; voir EP-A1-449 034) et de bromure d'éthyle (12 mmole). On a obtenu le propanol susmentionné avec un rendement de 87% (2 isomères en - OH 3:7).

Données analytiques :

| | | |
|---|---|---|
| IR : | 3380, 2960, 1450, 1130, 960 $cm^{-1}$ | |
| RMN($^1$H,360 MHz) : | 0,94(m,5H) ; 1,0-2,4(plusieurs m,13H) ; 1,68(s large, 3H) ; 3,8(m,1H, isomère mineur) ; 3,95 (m,1H, isomère majeur) ; 5,37 (m,1H) δ ppm | |
| SM : | isomère A (70%) : | 196($M^+$,3) ; m/e : 178(19), 163(32), 149(23), 135(100), 121(75), 107(72), 96(80), 81(88), 67(39), 55(58), 41(65) |
| | isomère B (30%) : | 196($M^+$,3) ; m/e : 178(20), 167(22), 163(35), 149(25), 135(93), 123 (76), 107(75), 96(80), 81(100), 69(66), 55(87), 41(82) |

d. cis-1-(2,2,6-triméthyl-3-méthylène-1-cyclohexyl)-3-butén-1-ol

Préparé à partir de 2,2,6-triméthyl-3-méthylène-1-cyclohexane-carbaldéhyde (6 g; 0,036 mole ; cis:trans 9:1 ; voir EP-A1-449 034)) et de chlorure d'allyle. On a obtenu le buténol susmentionné avec un rendement de 77% (pureté: 80%; 2 isomères 4:6).

Données analytiques :

| | | |
|---|---|---|
| IR : | 3550, 2950, 1640, 1450, 1380, 1020, 910 $cm^{-1}$ | |
| RMN($^1H$,360 MHz) : | 1,18(d,J=5Hz,3H) ; 1,21(s,3H) ; 1,25(s,3H) ; 1,2-2,5(plusieurs m,7H); 3,96(m,1H, isomère mineur); 4,12(m,1H, isomère majeur); 4,74(s,1H, isomère majeur) ; 4,77(s,1H, isomère majeur) ; 4,83(s,1H, isomère mineur) ; 4,85(s,1H, isomère mineur) ; 5,08(m,2H) ; 5,8(m,1H) δ ppm | |
| SM : | isomère A (60%) : | 208($M^+$, 0) ; 190(2), 175(8), 167(8), 149(21), 123(48), 107(42), 95 (44), 81(60), 69(50), 55(66), 41(100) |
| | isomère B (40%) : | 208($M^+$, 0) ; 190(3), 175(6), 167(35), 149(28), 123(48), 107(42), 95 (41), 81(55), 69(47), 55(68), 41(100) |

e. cis-1-(2,2,3,6-tétraméthyl-3-cyclohexén-1-yl)-3-butén-1-ol

Préparé à partir de 2,2,3,6-tétraméthyl-3-cyclohexène-1-carbaldéhyde (36 mmole ; voir sous c.) et de chlorure d'allyle (50 mmole). On a obtenu le buténol susmentionné avec un rendement de 86% (2 isomères 3:7).

Données analytiques :

P. éb. 70-75°/2 Pa

| | | |
|---|---|---|
| IR : | 3460, 2940, 1630, 1440 $cm^{-1}$ | |
| RMN($^1H$,360 MHz) : | 0,9-1,3(m,9H) ; 1,68(s large,3H) ; 1,4-2,5(m,6H) ; 3,97(m, 1H, isomère mineur) ; 4,09(m, 1H, isomère mineur) ; 5,11(m,2H) ; 5,36(m,1H) ; 5,83(m,1H) δ ppm | |
| SM : | isomère A (70%) : | 208($M^+$, 0) ; 190(3), 175(5), 167(6), 149(48), 133(23), 121(50), 107 (100), 95(48), 81(65), 71(42), 55(53), 41(97) |
| | isomère B (30%) : | 208($M^+$, 0); 190(3), 175(6), 167(18), 149(53), 123(73), 107(100), 95 (58), 81(72), 69(49), 55(57), 41(100) |

f. cis-1-(2,2,3,6-tétraméthyl-3-cyclohexén-1-yl)-1-butanol

Préparé de façon similaire à celle décrite sous c. mais en utilisant du bromure de propyle (rend. 82% ; 2 isomères 3:7).

g. 2-méthyl-1-2,2,c-3,t-6-tétraméthyl-r-1-cyclohexyl)-3-butén-1-ol

Préparé à partir de 2,2,c-3,t-6-tétraméthyl-r-1-cyclohexanecarbaldéhyde (18,0 g; 107 mmole ; voir sous a.) et de chlorure de chrotyle dans le THF (rend. 79%). Le produit a été purifié par chromatographie (180 g de $SiO_2$; toluène/éther acétique 95:5) pour obtenir une pureté de 97%.

Données analytiques :

P. éb. 56°/2,6 Pa

| | |
|---|---|
| IR : | 3550 $cm^{-1}$ ; - OH |
| RMN($^1H$,360 MHz) : | 0,78(s,3H) ; 0,84(d,J=7Hz,3H) ; 0,90(s,3H) ; 0,99(d,J=7Hz, 3H) ; 1,02(d,J=7Hz,3H) ; 2,47 (m,1H) ; 3,52(d,J=9Hz,1H) ; 5,14(m,2H) ; 5,67(m,1H) δ ppm |
| SM : | 224($M^+$,0) ; m/e : 169(25), 151(60), 139(8), 125(18), 109(34), 95(100), 83(95), 69(82), 55 (80), 43(68) |
| Odeur : | boisée, ambrée, urine |

h. 2-méthyl-1-(2,2,c-3,t-6-tétraméthyl-r-1-cyclohexyl)-1-propanol

Préparé à partir de 2,2,c-3,t-6-tétraméthyl-r-1-cyclohexanecarbaldéhyde (9,0 g; 54 mmole ; voir sous a.) et de bromure d'isopropyle dans le THF (rend. 45%). La distillation sur colonne Vigreux a fourni le produit pur à 86%.

Données analytiques :

P. éb. 74°/78 Pa

| | |
|---|---|
| IR : | 3550 $cm^{-1}$ ; - OH |
| RMN($^1H$,360 MHz) : | 0,78(s,3H) ; 0,83(d,J=7Hz,3H) ; 0,88(d,J=7Hz,3H) ; 0,89(s, 3H) ; 0,99(d,J=7Hz,3H) ; 1,0(d, J=7Hz,3H) ; 3,42(d,J=10Hz,1H) δ ppm |
| SM : | 212($M^+$,0) ; m/e : 169(45), 151(58), 125(60), 109(27), 95(78), 83(100), 69(71), 55(75), 43 (55) |
| Odeur : | boisée, transpiration, légèrement cyste, ambrée |

i. 1-(2,2,c-3,t-6-tétraméthyl-r-1-cyclohexyl)-1-butanol

Préparé à partir de 2,2,c-3,t-6-tétraméthyl-r-1-cyclohexanecarbaldéhyde (9,0 g ; 54 mmole ; voir sous a.) et de bromure de propyle dans le THF. La distillation sur colonne Vigreux a fourni le butanol susmentionné (rend. 57% ; 9% isomère t-3,c-6 et 4% isomère t-3,t-6). Le produit a été purifié par chromatographie (pureté 98%).
Données analytiques :
P. éb. 94°/2,2 x $10^2$ Pa

| | |
|---|---|
| IR : | 3450 $cm^{-1}$ ; - OH |
| RMN($^1$H,360 MHz) : | 0,77(s,3H) ; 0,83(d,J=7Hz,3H) ; 0,88(s,3H) ; 0,94(t,J=7Hz, 3H) ; 1,02(d,J=7Hz,3H) ; 3,94 (dxd,$J_1$=4, $J_2$=7Hz,1H) δ ppm |
| SM : | 212($M^+$,0) ; m/e : 169(10), 151(13), 140(18), 125(71), 109(10), 95(28), 83(100), 73(21), 69 (62), 55(75), 41(38) |
| Odeur : | boisée, résineuse |

j. <u>2-méthyl-1-(2,2,c-3,t-6-tétraméthyl-r-1-cyclohexyl)-1-butanol</u>
Dans un ballon de 250 ml à 1 col muni d'une tête à hydrogénation, on a placé le mélange de 7,0 g (31 mmole) de l'alcool décrit dans l'Exemple 1g., de 70 ml d'éther acétique et de 500 mg de Pd/C 5%. On a hydrogéné 30 min. à température ambiante pour absorber 750 ml d'hydrogène (prise théorique d'hydrogène = 700 ml). On a filtré sur célite et concentré : 8,45 g. On a distillé finement à la colonne à bande tournante pour isoler 5,85 g d'alcool désiré pur à 89% (rend. 74% ; mélange contenant 19% d'isomère t-3,t-6).
Données analytiques :
P. éb. 53°/1,5 Pa

| | |
|---|---|
| IR : | 3550 $cm^{-1}$ ; - OH |
| RMN($^1$H,360 MHz) : | 0,78(s,3H) ; 0,84(d,J=7Hz,6H) ; 0,89(s,3H) ; 0,92(t,J=7Hz, 3H) ; 0,99(d,J=7Hz,3H) ; 3,54(d, J=11Hz,1H) δ ppm |
| SM : | 226($M^+$,0) ; m/e : 169(22), 151(41), 125(38), 109(22), 95(65), 83(100), 69(75), 57(50), 41 (45) |
| Odeur : | boisée, terreuse, aqueuse, paille |

k. <u>1-(2,2,c-3,t-6-tétraméthyl-r-1-cyclohexyl)-1-pentanol</u>
Préparé à partir de 2,2,c-3,t-6-tétraméthyl-r-1-cyclohexanecarbaldéhyde (9,0 g ; 54 mmole ; voir sous a.) et de 1-bromobutane, dans le THF. Le produit a été purifié par chromatographie (pureté 98% ; mélange contenant 10% d'autres isomères). Rend. 53%.
Données analytiques :
P. éb. 78°/46 Pa

| | |
|---|---|
| IR : | 3470 $cm^{-1}$ ; - OH |
| RMN ($^1$H,360 MHz) : | 0,77(s,3H) ; 0,83(d,J=7Hz,3H) ; 0,88(s,3H) ; 0,92(t,J=7Hz, 3H) ; 1,01(d,J=7Hz,3H) ; 3,92 (dxd,$J_1$=4,$J_2$=7Hz,1H) δ ppm |
| SM : | 226($M^+$,0) ; m/e : 169(8), 151(15), 140(18), 125(81), 109(10), 95(28), 83(100), 69(90), 55 (55), 41(50) |
| Odeur : | boisée, cèdre |

l. <u>1-(2,2,c-3,t-6-tétraméthyl-r-1-cyclohexyl)-1-éthanol</u>
Préparé à partir de 2,2,c-3,t-6-tétraméthyl-r-1-cyclohexanecarbaldéhyde (42,0 g ; 0,25 mole ; voir sous a.) et iodure de méthyle, dans l'éther. Après distillation à la colonne à bande tournante et purification par chromatographie, on a obtenu le produit pur à 96% (rend. 78% ; mélange contenant 11% de l'isomère t-3,t-6).
Données analytiques :

| | |
|---|---|
| IR : | 3450 $cm^{-1}$ ; - OH |
| RMN($^1$H,360 MHz) : | 0,77(s,3H) ; 0,83(d,J=7Hz, 3H) ; 0,89(s,3H) ; 1,06(d,J=7Hz, 3H) ; 1,11(d,J=7Hz,3H) ; 4,18 (q,J=7Hz,1H) δ ppm |
| SM : | 184($M^+$,0) ; m/e : 140(8), 125(23), 109(5), 97(15), 83(100), 69(58), 55(42), 43(25) |
| Odeur : | rhubarbe, verte, fruitée |

m. <u>1-(2,2,c-3,t-6-tétraméthyl-r-1-cyclohexyl)-3-butén-1-ol</u>
Préparé à partir de 2,2,c-3,t-6-tétraméthyl-r-1-cyclohexanecarbaldéhyde (12 g ; 0,07 mole ; voir sous a.) et chlorure d'allyle (6,6 g ; 0,085 mole). Le produit a été purifié par chromatographie (pureté 98%). Rend. 82%.

Données analytiques :
P. éb. 35°/3 Pa

| | |
|---|---|
| IR : | 3500 $cm^{-1}$ ; - OH |
| RMN ($^1H$,360 MHz) : | 0,78(s,3H) ; 0,83(d,J~7Hz,3H) ; 0,89(s,3H) ; 1,04(d,J~7Hz, 3H) ; 2,2-2,5(m,2H) ; 3,98(m, 1H) ; 5,12(m,2H) ; 5,82(m,1H) δ ppm |
| SM : | 210($M^+$,0) ; m/e : 169(33), 151(58), 125(22), 109(34), 95(100), 83(97), 69(98), 55(98), 41 (98) |
| Odeur : | boisée, camphrée |

n. <u>3-méthyl-1-(2,2,c-3,t-6-tétraméthyl-r-1-cyclohexyl)-3-butén-1-ol</u>
Préparé à partir de 2,2,c-3,t-6-tétraméthyl-r-1-cyclohexanecarbaldéhydè (30,25 g; 0,18 mole ; voir sous a.) et de chlorure de méthallyle. Le produit a été distillé sur colonne Vigreux (rend. 88%) et purifié par chromatographie ($SiO_2$; éther de pétrole/éther éthylique 20:1) et ensuite sur bande tournante pour fournir le buténol susmentionné pur à 98%.
Données analytiques :
P. éb. 42°/3 Pa

| | |
|---|---|
| IR : | 1640, 3070 $cm^{-1}$ ; - C = $CH_2$ <br> 3520 $cm^{-1}$ ; - OH |
| RMN($^1H$,360 MHz) : | 0,80(s,3H) ; 0,83(d,J=7Hz,3H) ; 0,90(s,3H) ; 1,04(d,J=7Hz, 3H) ; 1,79(s,3H) ; 2,09(dxd, $J_1$=4,$J_2$=13Hz,1H) ; 2,48(dxd, $J_1$=4,$J_2$=13Hz, 1H) ; 4,05(m,1H) ; 4,78(s large,1H) ; 4,87(s large,1H) δ ppm |
| SM : | 224($M^+$,0) ; m/e : 169(32), 151(52), 139(24), 125(13), 109(38), 95(100), 83(82), 71(79), 55 (67), 41(62) |
| Odeur : | boisée, ambrée |

o. <u>trans-1-(2,2,6-triméthyl-3-méthylène-1-cyclohexyl)-3-butén-1-ol</u>
Préparé de façon identique à celle décrite sous d. mais en utilisant le 2,2,6-triméthyl-3-méthylène-1-cyclohexanecarbaldéhyde sous sa forme essentiellement trans (4,8 g; 0,029 mole, trans:cis > 95:5). On a obtenu le buténol susmentionné avec un rendement de 83% (pureté > 98% ; 2 isomères 1:1).

| | |
|---|---|
| IR : | 3500, 2930, 2250, 1640, 1460, 1216, 921 $cm^{-1}$ |
| RMN($^1H$,360 MHz,mélange de deux isomères) : | 0,97, 1,05, 1,07, 1,09, 1,1 et 1,15(6s,9H) ; 1,15(m,1H) ; 1,79(m,2H) ; 1,90-2,60(m,6H) ; 4,0(m,1H) ; 4,43(m,2H) ; 5,13(m,2H) ; 5,87(m,1H) δ ppm |
| RMN($^{13}C$) : | 157,9 et 157,8(q) ; 136,6 et 135,8(d) ; 177,8 et 117,7(t) ; 105,4 et 105(t) ; 71,8 et 70,2(d) ; 58,4 et 56,8(d) ; 44,4 et 44,0(t) ; 39,8 et 39,1(s) ; 38,5 et 37,8(t) ; 37,7(t) ; 33,04 et 32,99(t) ; 29,6 et 29,2(d) ; 26,9, 29,2, 26,6, 23,8, 22,7 et 22,4(q) δ ppm |
| SM : | 208($M^+$,0) ; m/e : 175(10), 167(14), 149(46), 138(32), 123 (52), 107(66), 95(64), 81(72), 67(50), 55(60), 41(100), 27 (22) |

L'aldéhyde de départ a été préparé par épimérisation de son isomère de configuration cis (voir sous d.) comme suit.

Dans un ballon tricol de 250 ml, équipé d'un réfrigérant, d'un thermomètre et d'une ampoule d'introduction, placé sous atmosphère de $N_2$, on a introduit le cis-2,2,6-triméthyl-3-méthylène-1-cyclohexanecarbaldéyhde (6,4 g ; 0,75 mole), 70 ml d'éther sulfurique et 3 ml de méthylate de sodium (5,6 M). On a chauffé pendant 15 h à reflux.

Un contrôle chromatographique a indiqué la présence de 2 pics (trans:cis 88:12).

On a refroidi, repris à l'éther, lavé jusqu'à neutralité avec de la saumure, séché sur $Na_2SO_4$, filtré et concentré sous vide. Après distillation au four à boules (120°/3x$10^2$ Pa), on a obtenu 5,3 g de l'aldéhyde désiré sous forme d'une huile incolore (rend. : 88% ; rapport trans:cis 88:12).

Cette huile a été purifiée encore sur colonne Carbowax de 5 m pour fournir le trans-2,2,6-triméthyl-3-méthylène-1-cyclohexanecarbaldéhyde avec une pureté d'au moins 95% et les caractères analytiques suivants :

| | |
|---|---|
| IR : | 2965, 2254, 1719, 1640, 1457, 908 $cm^{-1}$ |
| RMN($^1H$,360 MHz) : | 0,83(d,J=6Hz,3H); 1,05(m,1H); 1,16(s,3H); 1,18(s,3H); 1,69(dd,J=4Hz, 9Hz,1H); 1,89(m,1H); |

2,2(m,2H); 2,37(m,1H); 4,68(s large,2H); 9,7(d,J=4Hz,1H) à ppm

RMN($^{13}C$): 206,4(d) 154,9(s) 106,1(t); 66,5(d); 38,7(s); 35,9(t) 32,4(t); 28,3(d); 26,6(q); 22,6(q); 20,3(q) δ ppm

SM : 166($M^+$,6); m/e: 151(34), 135(72), 123(41), 109(38), 95(100), 81(86), 67(61), 55(50), 41(76), 29(39)

Exemple 2

Préparation de cétones de formule (I)

Méthode générale des réactions d'oxydation d'alcools (II)

Dans un ballon tricol, on introduit ~1,5 éq./M de chlorochromate de pyridinium (PCC) en suspension dans le dichlorométhane et célite (1 éq./g de PCC). On ajoute ensuite rapidement et sous forte agitation ~ 1 éq./M d'alcool. On laisse réagir à température ambiante jusqu'à oxydation complète. On ajoute de l'éther et on filtre. On traite le produit obtenu de la façon usuelle.

On a préparé les cétones suivantes:

a. 1-(2,2,c-3,t-6-tétraméthyl-r-1-cyclohexyl)-4-pentén-1-one

Préparée par oxydation de l'alcool décrit dans l'Exemple la. (2,7g ; 0,012 mole). Réaction pendant 1 nuit et distillation sur résidu (rend. ~90% ; pureté 99% ; mélange de 2 isomères : c-3,t-6/t-3,c-6 75:24).

Données analytiques:

IR: 1640, 3080(C = $CH_2$); 1700 (> C = O) $cm^{-1}$

RMN($^1H$,360 MHz) :
c-3,t-6: 0,74(d,3H,J=7Hz); 0,81(d,3H,J=7Hz) ; 0,82(s,3H) ; 0,90(s,3H) ; 2,05(d,1H, J=11Hz); 4,97(d,1H,J=9Hz); 5,04(d,1H,J=16Hz); 5,82(m,1H) δ ppm
t-3,c-6: 0,74(d,3H,J=7Hz); 0,82(s,3H) ; 0,84(d,3H,J=7Hz); 0,86(s,3H) ; 2,05(d,1H, J=11Hz);4,94(d,1H,J=9Hz); 5,04(d,1H,J=16Hz); 5,82(m,1H) δ ppm

SM :
c-3,t-6: 222($M^+$,2); m/e: 167(15), 139(47), 124(10), 97(12), 83(100), 69(45), 55(60), 41(20)
t-3,c-6 : 222($M^+$,3) ; m/e : 139(19), 124(64), 109(42), 97(11), 83(100), 69(60), 55(65), 41(28)

b. (E)-1-(2,2,c-3,t-6-tétraméthyl-r-1-cyclohexyl)-2-pentén-1-one

Préparée par isomérisation de la cétone décrite sous a. (1,4 g ; 0,006 mole) à l'aide de $RhCl_3.2H_2O$ (14 mg), dans 15 ml d'éthanol. On a chauffé à reflux pendant 24h, concentré et distillé au four à boules. Le produit ainsi obtenu contenait 30% de 3-éthoxy-1-(2,2,c-3,t-6-tétraméthyl-r-1-cyclohexyl)-1-pentanone. On lui a ajouté 10 ml de toluène et 0,2g d'acide p-toluènesulfonique. Après agitation pendant 45 min à reflux, on a concentré et distillé au four à boules pour obtenir 1,2g (rend. 85%) d'un mélange contenant la penténone désirée (77%) et son isomère t-3,t-6 (21%).

Données analytiques :

IR : 1620,1660,1680 $cm^{-1}$ (> C = O)

RMN($^1H$,360 MHz) : 0,72(d,J=7Hz,3H) ; 0,82(s,3H) ; 0,83(d,J=7Hz,3H) ; 0,88 (s,3H) ; 1,08(t,J=7Hz,3H) ; 2,24 (quint,J=7Hz,2H) ; 6,17(d,J=15Hz,1H) ; 6,85 (m,1H) δ ppm

SM : 222($M^+$,5) ; m/e : 193(12), 124(38), 109(23), 83(100), 69(24), 55(33), 41(14)

c. 1-(2,2,c-3,t-6-tétraméthyl-r-1-cyclohexyl)-1-propanone

Obtenue à partir de l'alcool décrit dans l'Exemple 1b. (3,25 g ; 16 mmole). Après distillation sur colonne Vigreux de 10 cm, on a obtenu la cétone désirée en mélange avec 9% de son isomère t-3,c-6 (rend. 65%).

Données analytiques :

P. éb. 81°/3,9x$10^2$ Pa

IR : 1710 $cm^{-1}$:

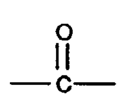

| | |
|---|---|
| RMN($^1$H,360 MHz) : | 0,73(d,J=7Hz,3H) ; 0,81(d,J=7Hz,3H) ; 0,82(s,3H) ; 0,88(s,3H) ; 1,01(t,J=7Hz,3H) ; 2,05(d, J=9Hz,1H) ; 2,32(m,1H) ; 2,55 (m,1H) δ ppm |
| SM : | 196(M$^+$,4) ; m/e : 167(12), 139(32), 124(13), 99(14), 83(100), 69(39), 57(52), 41(19) |

d. <u>cis-1-(2,2,3,6-tétraméthyl-3-cyclohexén-1-yl)-1-propanone</u>
Préparée à partir de l'alcool décrit dans l'Exemple 1c. (7 mmole). Pureté : 95% ; rend. 80%.
Données analytiques :

| | |
|---|---|
| IR : | 2950, 1700, 1450, 1370, 1100 cm$^{-1}$ |
| RMN($^1$H,360 MHz) : | 0,92(d,J=7Hz,3H) ; 0,95(s,3H) ; 0,99(t,J=7Hz,3H) ; 1,13(s,3H) ; 1,64(s large,3H) ; 1,94(m, 2H) ; 2,14(m,1H) ; 2,42(m,2H) ; 2,62(d,J=3,5Hz,1H) ; 5,4(s large,1H) δ ppm |
| RMN ($^{13}$C) : | 214,1(s) ; 137,3(s) ; 122,1(d) ; 63,0(d) ; 41,0(t) ; 37,4(s) ; 31,2(t) ; 30,1(q) ; 27,5(d) ; 24,6 (q) ; 20,0(q); 19,1(q) ; 7,2(q) δ ppm |
| SM: | 194(M$^+$,28) ; m/e : 137(37), 122(28), 112(31), 95(50), 83(62), 57(100) |

e. <u>cis-1-(2,2,6-ttiméthyl-3-méthylène-1-cyclohexyl)-3-butén-1-one</u>
Préparée à partir de l'alcool décrit dans l'Exemple 1d. (1,5 g ; 7,2 mmole).
Pureté: 80% ; rend. 97%.
Données analytiques :

| | |
|---|---|
| IR : | 3000, 2900, 1710, 1640, 1460, 1380, 1050, 910, 890 cm$^{-1}$ |
| RMN($^1$H,360 MHz) : | 0,88(d,J=7Hz,3H) ; 1,06(s,3H) ; 1,16(s,3H) ; 1,5(m,1H) ; 1,67(m,2H) ; 2,14(m,1H) ; 2,37(m, 1H) ; 2,64(d,J=7Hz,1H) ; 3,16(m,2H) ; 4,59(s,1H) ; 4,77(s,1H) ; 5,1(m,2H) ; 5,9(m,1H) δ ppm |
| SM : | 206(M$^+$,2) ; m/e : 165(24), 137(64), 123(36), 95(100), 81(85), 69(25), 55(19), 41(32) |

f. <u>cis,(E)-1-(2,2,6-triméthyl-3-méthylène-1-cyclohexyl)-2-butén-1-one</u>
Préparée par isomérisation de la cétone décrite sous e. (1,3 g ; 6,3 mmole) à l'aide d'acide acétique (5 gouttes). On a chauffé à 100°, sous agitation, 1 nuit. Après refroidissement, reprise à l'éther, lavage au $NaHCO_3$ sat., séchage sur $Na_2SO_4$, filtration et concentration sous vide, on a distillé au four à boules (10 Pa/150°C) pour obtenir la buténone susmentionnée (pureté: 80%, rend. 77%). Cette buténone a été encore purifiée par chromatographie (pure à 95%).
Données analytiques :

| | |
|---|---|
| IR : | 3000, 1680, 1630, 1440, 1070, 920 cm$^{-1}$ |
| RMN($^1$H,360 MHz) : | 0,83(d,J=7Hz,3H) ; 1,02(s,3H) ; 1,18(s,3H) ; 1,5(m,1H) ; 1,58(s,1H); 1,72(m,1H) ; 1,86(dd, J=2Hz,7Hz,3H) ; 2,57(m,1H) ; 2,34(m,1H) ; 2,79(d,J=7Hz,1H) ; 4,61(s,1H) ; 4,78(s,1H) ; 6,16(m,1H) ; 6,75(m,1H) δ ppm |
| RMN ($^{13}$C) : | 202,7(s) ; 152,9(s) ; 140,8(d), 135,2(d) ; 106,8(t) ; 61,7(d) ; 38,9(s) ; 32,3(t) ; 30,9(t) ; 30,9 (d) ; 29,2(q) ; 26,7(q); 19,8(q) ; 16,1(q) δ ppm |
| SM : | 206(M$^+$,5) ; m/e : 191(6), 163(4), 149(7), 137(11), 123(17), 109(21), 95(36), 81(31), 69(100), 55(17), 41(33) |

g. <u>cis-1-(2,2,3,6-tétraméthyl-3-cyclohexén-1-yl)-3-butén-1-one</u>
Préparée par oxydation de l'alcool décrit dans l'Exemple 1e. (7 mmole) avec 86% de rendement.
Données analytiques :

| | |
|---|---|
| IR : | 2940, 1680, 1440, 1370 cm$^{-1}$ |
| RMN($^1$H,360 MHz) : | 0,94(d,J=7Hz,3H) ; 0,97(s,3H) ; 1,12(s,3H) ; 1,64(s large, 3H) ; 1,94(m,2H) ; 2,15(m,1H) ; 2,65(d,J=7Hz,1H) ; 3,18(m,2H) ; 5,07(dd, J=1Hz,16Hz,1H) ; 5,15(dd,J=1Hz,10Hz,1H) ; 5,4 (s large,1H) ; 5,92 (m,1H) δ ppm |
| SM : | 206(M$^+$,4) ; m/e : 165(29), 137(100), 109(29), 95(99), 81(76), 69(49), 57(65), 41(36) |

h. <u>cis(E)-1-(2,2,3,6-tétraméthyl-3-cyclohexén-1-yl)-2-butén-1-one</u>
Préparée par isomérisation de la cétone décrite sous g. (1 g; 5,3 mmole) à l'aide d'acide acétique (5 gouttes), en chauffant à 50° pendant 5 h. Après le traitement usuel, distillation au four à boules (150°/10 Pa) et purification par chromatographie ("flash", cyclohexane/éther acétique 95:5), on a obtenu la buténone susmentionnée pure à 98% (rend. 84%).

Données analytiques :

IR : 2990, 1680, 1620, 1440, 1070, 980 $cm^{-1}$
RMN($^1H$,360 MHz): 0,90(d,J=7Hz,3H) ; 0,95(s,3H) ; 1,14 (s,3H) ; 1,67(s large,3H) ; 1,86(dd,J=1Hz,7Hz,3H) ; 1,93(m,2H) ; 2,18(m, 1H) ; 2,75(d,J=7Hz,1H) ; 5,43(s large,1H) ; 6,22(dd,J=1Hz, 16Hz, 1H) ; 6,78 (m,1H) δ ppm
RMN ($^{13}C$) : 202,8(s) ; 140,9(d) ; 138,1(s) ; 134,6(d) ; 122,0(d) ; 61,1(d) ; 37,3(s) ; 31,4(t) ; 30,2(q) ; 27,7 (d) ; 24,8(q) ; 20,0(q) ; 19,0(q) ; 18,1(q) δ ppm
SM : 206($M^+$,4) ; m/e : 191(3), 135(13), 123(27), 109(40), 95(38), 69(100), 41(22)
Odeur : damasconée, résineuse, balsamique, épicée

i. cis-1-(2,2,3,6-tétraméthyl-3-cyclohexén-1-yl)-1-butanone
Préparée par oxydation de l'alcool décrit dans l'Exemple 1f. (7 mmole). La butanone désirée a été obtenue avec un rendement de 88% et purifiée par chromatographie (pureté: 96%).
Données analytiques :

IR : 2940,1740,1470,1350 $cm^{-1}$
RMN($^1H$,360 MHz) : 0,90(t,J=7Hz,3H) ; 0,91(d,J=7Hz,3H) ; 0,96(s,3H) ; 1,12(s, 3H) ; 1,55(m,2H) ; 1,63(s large, 3H) ; 1,94(m,2H) ; 2,13(m,1H) ; 2,39(dt, J=3,5Hz, 7Hz,2H) ; 2,59(d,J=3Hz,1H) ; 5,4(s large, 1H) δ ppm
RMN ($^{13}C$) : 213,5(s) ; 137,4(s) ; 122,1(d) ; 62,8(d) ; 50,1(t) ; 37,5(s) ; 31,2(t) ; 30,1(q) ; 27,7(d) ; 24,6 (q) ; 20,0(q) ; 19,1(q) ; 16,4(t) ; 13,8(q) δ ppm
SM : 208($M^+$,30) ; m/e : 193(8), 165(15), 137(63), 95(67), 83(72), 71(100), 43(83)
Odeur : fruitée, florale, aromatique, camphrée, celluloïde

j. 2-méthyl-1-(2,2,c-3,t-6-tétraméthyl-r-1-cyclohexyl)-3-butén-1-one
Préparée par oxydation de l'alcool décrit dans l'Exemple 1g. (10,5 g ; 47 mmole) avec 83% de rendement. La distillation à la colonne à bande tournante a fourni la buténone désirée pure à 98%.
Données analytiques :
P. éb. 43°/1,5 Pa

IR : 1750 $cm^{-1}$ :

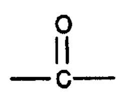

RMN($^1H$,360 MHz) : 0,64(d,J=7Hz,3H) ; 0,76(s,3H) ; 0,82(d,J=7Hz,3H) ; 0,95(s,3H) ; 1,15(d,J=7Hz,3H) ; 2,3(d, J=10Hz,1H) ; 3,3(m,1H) ; 5,14(d,J=9Hz,1H) ; 5,2(d,J=16Hz,1H) ; 5,55(m,1H) δ ppm
SM : 222($M^+$,0) ; m/e : 167(5), 139(22), 97(12), 83(100), 69(42), 55(40), 41(12)
Odeur : florale, poudreuse, balsamique, myrrhe

k. 2-méthyl-1-(2,2,c-3,t-6-tétraméthyl-r-1-cyclohexyl)-1-propanone
Préparée par oxydation de l'alcool décrit dans l'Exemple 1h. (4,4 g ; 21 mmole). La distillation sur colonne Vigreux a fourni un produit pur à 94% (8% d'isomère t-3,c-6 ; rend. 56%).
Données analytiques :
P. éb. 72°/1,2x$10^2$ Pa

IR : 1700 $cm^{-1}$ :

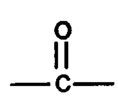

RMN($^1H$,360 MHz) : 0,73(d,J=7Hz,3H) ; 0,77(s,3H) ; 0,82(d,J=7Hz,3H) ; 0,92(s,3H); 1,04(d,J=7Hz,3H) ; 1,06(d, J=7Hz,3H) ; 2,22(d,J=10Hz,1H) ; 2,66(hept,J=7Hz,1H) δ ppm

SM : $210(M^+,3)$ ; m/e : 167(19), 139(52), 97(15), 83(100), 69(43), 55(32), 43(35)
Odeur : labdanum, oppoponax, myrrhe, violette, damasconée, herbacée, menthée, tabac

l. 1-(2,2,c-3,t-6-tétraméthyl-r-1-cyclohexyl)-1-butanone

Préparée par oxydation de l'alcool décrit dans l'Exemple 1i. (5,45 g ; 26 mmole). Le produit a été purifié par chromatographie (pureté 99% ; 10% isomère t-3,c-6). Rend. 47%.

Données analytiques :

P. éb. 65°/85 Pa

IR : 1710 $cm^{-1}$:

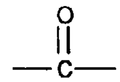

RMN($^1H$,360 MHz) : 0,74(d,J=7Hz,3H) ; 0,81(d,J=7Hz,3H) ; 0,82(s,3H) ; 0,9(s,3H) ; 0,92(t,J=7Hz,3H) ; 2,03(d, J=10Hz,1H) ; 2,3(m,1H) ; 2,5(m,1H) δ ppm
SM : $210(M^+,5)$ ; m/e : 139(15), 124(75), 113(35), 109(25), 97(12), 83(100), 69(42), 55(38), 43 (39)
Odeur : boisée, élégante, poudrée

m. 2-méthyl-1-(2,2,c-3,t-6-tétraméthyl-r-1-cyclohexyl)-1-butanone

Préparée par oxydation de l'alcool décrit dans l'Exemple 1j. (4,05 g ; 18 mmole). Après distillation au four à boules (80°/1,8 Pa), on a obtenu le produit pur à 99% (mélange contenant 10% d'isomère t-3,t-6). Rend. 96%.

Données analytiques :

IR : 1750 $cm^{-1}$ :

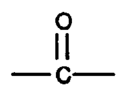

RMN($^1H$,360 MHz) : 0,72(d,J=7Hz,3H) ; 0,75(s,3H) ; 0,82(d,J=Hz,3H) ; 0,93(s,3H) ; 0,94(t,J=7Hz,3H) ; 1,02(d, J=7Hz,3H) ; 2,21(d,J=10Hz,1H) ; 2,49(m,1H) δ ppm
SM : $224(M^+,2)$ ; m/e : 167(10), 139(33), 97(10), 83(100), 69(34), 57(40), 41(15)
Odeur : ironée, boisée, terreuse, iris

n. 1-(2,2,c-3,t-6-tétraméthyl-r-1-cyclohexyl)-1-pentanone

Préparée par oxydation de l'alcool décrit dans l'Exemple 1k. (5,25 g ; 23 mmole). Après purification, on a obtenu le produit pur à 99% (10% d'isomère t-3,c-6 ; rend. 75%).

Données analytiques :

P. éb. 73°/50 Pa

IR : 1710 $cm^{-1}$ :

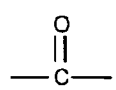

RMN($^1H$,360 MHz) : 0,74(d,J=7Hz,3H) ; 0,81(d,J=7Hz,3H) ; 0,82(s,3H) ; 0,89(s, 3H) ; 0,90(t,J=7Hz,3H) ; 2,03(d, J=10Hz,1H) ; 2,32(m,1H) ; 2,52(m,1H) δ ppm
SM : 224(M+,5) ; m/e : 167(10), 139(46), 124(16), 97(15), 83(100), 69(42), 57(55), 41(28)
Odeur : boisée, douce

o. 1-(2,2,3,6-tétraméthyl-1-cyclohexyl)-3-butén-1-one

Préparée par oxydation de l'alcool décrit dans l'Exemple 1m. (6,5 g; 0,03 mole). Le produit obtenu est un mélange contenant 40% d'isomère r-1, c-3, t-6, 30% d'isomère r-1, t-3, c-6 et 20% d'isomère r-1, t-3, t-6 (rend. 75%).
Données analytiques:
P. éb. 55°/5 Pa

IR: 1700 $cm^{-1}$; > C = O
RMN($^1H$,360 MHz) : c-3,t-6: 0,75(d,J=7Hz,3H) ; 0,82(s,3H) ; 0,86(d,J=7Hz,3H) ; 0,93(s,3H) ; 2,09(d, J=11Hz,1H) ; 3,20(m,2H) ; 5,08(d,J=18Hz,1H) ; 5,16(d,J=8Hz,1H) ; 5,92 (m, 1H) δ ppm
t-3, c-6 : 0,74(d,J=7Hz,3H); 0,81(s,3H) ; 0,87(d,J=7Hz,3H) ; 0,88(s,3H); 2,53(d,J=-4Hz) ; 3,2(m,2H); 5,06(d,J=18Hz,1H) ; 5,16(d,J=8Hz, 1H) ; 5,92(m,1H) δ ppm

SM : c-3,t-6: 208($M^+$,0) ; m/e : 167(16), 139(37), 97(13), 83(100), 69(53), 55(23), 41(22)
t-3,c-6 : 208($M^+$,0) ; m/e : 167(12), 139(33), 97(13), 83(100), 69(64), 55(35), 41(28)
t-3,t-6: 208($M^+$,11); m/e 124(68), 11(49), 97(10), 83(69), 69(100), 55(28), 41(27)

Odeur : florale, camphrée, poussière

p. (E)-1-(2,2,c-3,t-6-tétraméthyl-r-1-cyclohexyl)-2-butén-1-one
Dans un ballon muni d'un réfrigérant, on a chargé 2,3 g (0,011 mole) de la cétone décrite sous o., 1,5 ml d'une solution à 15% de HCl et 23 ml de THF. On a chauffé à reflux pendant 48 h. On a repris à l'éther, décanté le HCl, lavé 1 fois à l'eau et 1 fois avec $NaHCO_3$, ensuite de nouveau à l'eau jusqu'à neutralité.
On a distillé sur résidu pour obtenir un produit (rend. 82%; pureté 97%) sous forme d'un mélange contenant 74% de l'isomère susmentionné, 21% de l'isomère t-3,c-6 et 2% de l'isomère t-3,t-6.
Données analytiques :

IR: 1620, 1640, 1680 $cm^{-1}$ :

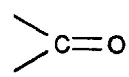

RMN($^1H$,360 MHz) : c-3,t-6: 0,72 (d,3H,J=7Hz) ; 0,82(s,3H) ; 0,83(d,3H,J=7Hz) ; 0,87(s,3H); 1,89(d,3H, J=7Hz) ; 6,2(d,1H,J=16Hz) ; 6,8(m,1H) δ ppm
t-3,c-6 : 0,75(d,3H,J=7Hz); 0,79(s,3H) ; 0,83(d,3H,J=7Hz) ; 0,89(s,3H) ; 1,88(d,3H, J=7Hz); 2,73(d,1H,J=4Hz) ; 6,14(d,J=16Hz,1H) ; 6,73(m,1H) à ppm

SM : t-3,t-6: 208($M^+$,7) m/e 137(19), 124(57), 111(32), 95(15), 83(73), 79(12), 69(100), 55(43), 41 (44)
t-3,c-6 : 208($M^+$,5); m/e: 193(10), 124(68), 109(63), 83(28), 69(100), 55(42), 41(43)
c-3,t-6: 208($M^+$,3); m/e : 124(37), 111(29), 83(46), 69(100), 55(31), 41(30)

Odeur : florale, fruitée, rosée

q. trans-1-(2,2,6-triméthyl-3-méthylène-1-cyclohexyl)-3-butén-1-one
Préparée par oxydation de l'alcool décrit dans l'Exemple 1o. (5 g; 0,024 mole). Après "flash" chromatographie ($SiO_2$; cyclohexane/éther 8:2), on a obtenu la cétone désirée pure à 97%.

IR : 2931, 2254,1706, 1640, 1463, 1384, 1216, 908 $cm^{-1}$
RMN($^1H$,360 MHz) : 0,77(d,J=6Hz,3H) ; 1,05(m,2H) ; 1,08(s,3H) ; 1,12(s,3H) ; 1,72(m,1H) ; 2,12(m,2H) ; 2,39 (m,1H) ; 3,2(m,2H) ; 4,65(m,2H) ; 5,14(m,2H) ; 5,93(m,1H) à ppm
RMN($^{13}C$) : 212,2(s) ; 155,8(s) ; 130,6(d) ; 118,5(t) ; 105,3(t) ; 66,1(d) ; 52,3(t) ; 39,2(s); 36,3(t); 32,2(t); 31,0(d); 27,2(q); 22,2(q); 20,5(q) δ ppm
SM : 206($M^+$,0) ; m/e : 165(33), 137(100), 123(8), 95(98), 81(61), 67(18), 55(16), 41(37)

r. trans-(E)-1-(2,2,6-triméthyl-3-méthylène-1-cyclohexyl)-2-butén-1-one
Préparée par isomérisation de la cétone décrite sous q. On a chauffé, à 100°, pendant 3 h, sous azote, 2,5 g (0,012 mole) de ladite cétone en présence de 6 gouttes d'acide acétique (on a observé la disparition complète du

produit de départ).
On a laissé refroidir et purifié par chromatographie ($SiO_2$; toluène) pour obtenir 0,7 g de trans(E)-1-(2,2,6-triméthyl-3-méthylène-1-cyclohexyl)-2-butén-1-one sous forme de cristaux blancs (rend. 40%).
Pureté : 97%
P. f. 35°-36°

| | |
|---|---|
| IR : | 2968, 2253, 1706, 1623, 1462, 1383, 1216, 906 $cm^{-1}$ |
| RMN($^1$H,360 MHz) : | 0,74(d,J=6Hz,3H); ; 1,05(m,1H) ; 1,06(s,3H) ; 1,08(s,3H); ; 1,85(m,1H) ; 1,89(dd,J=2Hz, 6Hz,3H); ; 2,15(m,2H) ; 2,36(m,2H) ; 4,65(s large,2H) ; 6,18(m,1H) ; 6,82(m,1H) δ ppm |
| RMN($^{13}$C) : | 156,3(s) ; 141,2(d) ; 135(d) ; 105,2(t) ; 63,8(d) ; 39,3(s) ; 36,5(t) ; 39,4(t) ; 30,9(d) ; 27,3(q) ; 22,5(q) ; 20,5(q) ; 18,2(q) δ ppm |
| SM : | 206($M^+$,12) ; m/e : 191(14), 163(12), 149(12), 137(30), 123(20), 109(22), 95(44), 81(36), 69(100), 55(20), 41(59) |
| Odeur : | verte, camphrée, oseille, piquante |

s. trans-(E)-1-(2,2,3,6-tétraméthyl-3-cyclohexén-1-yl)-2-butén-1-one
On a chauffé à reflux dans le toluène (5 ml) pendant 3 h, en présence de 10 mg d'acide p-toluènesulfonique et sous $N_2$, 0,18 g (4,8 mmole) de la cétone décrite sous r. Après le traitement usuel et distillation au four à boules (150°/10 Pa), on a obtenu 0,17g de la buténone désirée sous forme d'une huile jaune paille (rend. 92%; pureté 94%).

| | |
|---|---|
| IR : | 2970, 1710, 1640, 1450, 1220, 910 $cm^{-1}$ |
| RMN($^1$H,360 MHz) : | 0,81(d,J=6Hz,3H) ; 1,01(s,3H) ; 1,02(s,3H) ; 1,64(s large,3H) ; 1,65(m,1H) ; 1,90(dd, J=2Hz, 6Hz,3H) ; 2,12(m,2H) ; 2,56(d,J=10Hz,1H) ; 5,28(m,1H) ; 6,23(m,1H) ; 6,83(m,1H) δ ppm |
| RMN($^{13}$C) : | 203,8(s) ; 144,5(d) ; 140,6(s) ; 134,7(d) ; 120,9(d) ; 61,7(d) ; 38,1(s) ; 34,6(t) ; 27,4(q) ; 27,0 (d) ; 22,8(q) ; 20,2(q) ; 18,6(q) ; 18,2(q) δ ppm |
| SM : | 206($M^+$,13) ; m/e : 191(12), 163(8), 149(8), 137(18), 122(31), 109(43), 95(49), 91(32), 81 (28), 69(100), 55(22), 41(83) |
| Odeur : | pomme, damasconée, verte, vineuse |

Exemple 3

Composition parfumante

On a préparé une composition parfumante de base pour un parfum de type féminin, en mélangeant les ingrédients suivants :

| Ingrédient | Parties en poids |
|---|---|
| Florol ® 1) | 200 |
| Acétate de benzyle | 40 |
| Acétate de styrallyle | 120 |
| Acétate de vétyvéryle | 560 |
| 10-Undécénal à 10%* | 80 |
| Aldéhyde hexylcinnamique | 80 |
| Ambrettolide 2) à 10%* | 100 |
| Allyle ionone à 1%* | 80 |
| γ-Undécalactone à 1%* | 160 |
| Cétone framboise à 1%* | 80 |
| Cassis Bourgeons absolue | 100 |
| Essence de coumarine à 10%* | 140 |

* dans le dipropylène glycol (DIPG)
1) tétrahydro-2-isobutyl-4-méthyl-4(2H)-pyranol; origine: Firmenich SA, Genève, Suisse
2) 7-hexadécén-16-olide ; origine : L. Givaudan, Genève, Suisse

(suite)

| Ingrédient | Parties en poids |
|---|---|
| Cyclosia ® 3) base | 500 |
| Essence de galbanum | 60 |
| Essence de clous de girofle | 20 |
| Isobutylquinoléine 4) à 10%* | 40 |
| Isobutyrate de phénoxyéthyle | 20 |
| Jasmin absolue | 240 |
| Levocitrol 5) | 440 |
| Lilial ® 6) | 40 |
| Ethyl linalol | 140 |
| Lyral ® 7) | 260 |
| Iralia ® 8) | 460 |
| Mousse de chêne absolue à 50%* | 320 |
| Hédione ® 9) | 1140 |
| Essence de néroli | 120 |
| Essence de patchouli | 40 |
| Alcool phényléthylique | 760 |
| Essence d'orange douce du Portugal | 80 |
| Polywood ® 10) | 160 |
| Rose absolue du Maroc | 120 |
| Salicylate de benzyle | 440 |
| Essence de santal | 260 |
| Stemone ® 11) | 60 |
| Tonalid ® 12) à 10%* | 130 |
| Vanilline à 1%* | 50 |
| Vertofix coeur 13) | 1640 |
| Ylang extra | 140 |
| Jacinthe absolue synthétique | 160 |
| Rose absolue synthétique | 320 |
| Total | 9900 |

* dans le dipropylène glycol (DIPG)

3) hydroxycitronellal; origine: Firmenich SA, Genève, Suisse

4) 6-(1-méthylpropyl)quinoléine; origine: International Flavors and Fragrances Inc, USA

5) levo-citronellol; origine: Firmenich SA, Genève, Suisse

6) 3-(4-tert-butyl-1-phényl)-2-méthylpropanal ; origine : L. Givaudan, Genève, Suisse

7) 4-(4-hydroxy-4-méthylpentyl)-3-cyclohexène-1-carboxaldéhyde; origine : International Flavors & Fragrances Inc, USA

8) méthylionone; origine: Firmenich SA, Genève, Suisse

9) dihydrojasmonate de méthyle; origine : Firmenich SA, Genève, Suisse

10) acétate de perhydro-5,5,8a-triméthyl-2-naphtyle ; origine: Firmenich SA, Genève, Suisse

11) 5-méthyl-3-heptanone oxime ; origine: L. Givaudan, Genève, Suisse

12) 7-acétyl-1,1,3,4,4,6-hexaméthyltétraline ; origine : PFW, Hollande

13) origine : International Flavors & Fragrances Inc., USA

A cette composition de base de type fleuri, rosé, jasminé, Chypre, on a ajouté 1% de (E)-1-(2,2,c-3,t-6-tétraméthyl-r-1-cyclohexyl)-2-pentén-1-one. On a ainsi obtenu une composition nouvelle dont la note fleurie était nettement renforcée, ayant pris un côté fleuri-damasconé, et qui développait maintenant une odeur beaucoup plus orientale, poudrée, et de plus avec un caractère myrrhe prononcé.

Cet effet iris, myrrhe très apprécié apparaissait aussi clairement lorsqu'on a ajouté à la composition de base la même quantité de 1-(2,2,c-3,t-6tétraméthyl-r-1-cyclohexyl)-1-propanone ou de cis-1-(2,2,3,6-tétraméthyl-3-cyclohexén-1-yl)-1-propanone, cette dernière impartissant en plus une connotation plus herbacée, fenchylique à la composition.

Exemple 4

Composition parfumante

On a préparé une composition parfumante de base à odeur fleurie, rosée par mélange des ingrédients suivants:

| Ingrédient | Parties en poids |
|---|---|
| Alcool cinnamique à 50% * | 100 |
| Allyle ionone à 10% * | 500 |
| Citronellol | 400 |
| Coumarine | 750 |
| Galaxolide ® 50 1) | 1500 |
| Géraniol | 500 |
| Essence de géranium Bourbon | 150 |
| Cyclosia ® base 2) | 600 |
| Héliotropine | 250 |
| Ethyl linalol | 500 |
| Lyral ® 3) | 500 |
| Musc cétone | 600 |
| Phénéthylol | 600 |
| Polywood ® 4) | 500 |
| Polysantol ® 5) à 50% * | 200 |
| Vanilline | 150 |
| Vertofix coeur 6) | 1200 |
| Total | 9000 |

* dans le DIPG

1) 1,3,4,6,7,8-hexahydro-4,6,6,7,8,8-hexaméthyl-cyclopenta[γ]isochromène ; origine : International Flavors and Fragrances Inc., USA

2), 3), 4), 6) voir Exemple 3

5) 3,3-diméthyl-5-(2,2,3-triméthyl-3-cyclopentén-1-yl)-4-pentén-2-ol ; origine : Firmenich SA, Genève, Suisse

L'addition de 1% en poids de (E)-1-(2,2,c-3,t-6-tétraméthyl-r-1-cyclohexyl)-2-pentén-1-one à cette composition de base a fourni une nouvelle composition dont la note fleurie, rosée ressortait beaucoup plus que dans la composition de base et possédait maintenant un joli côté fruité. D'autre part, la composition avait maintenant une connotation irisée, poudrée, myrrhe qui était absente avant l'adjonction du composé de l'invention.

Exemple 5 - 15

Parfumage d'articles divers

On a parfumé les articles indiqués ci-après en ajoutant de la (E)-1-(2,2,c-3,t-6-tétraméthyl-r-1-cyclohexyl)-2-pentén-1-one, dans les concentrations indiquées, aux bases appropriées non parfumées.

| | Article | Conc. (% en poids) | Odeur/aspect [25°C] | Odeur/aspect [40°C] |
|---|---|---|---|---|
| 5 | Eau de toilette alcoolique (alcool 95°) | 5.0 | S/N | S/N |
| 6 | Crème huile/eau | 0.4 | S/N | S/N |
| 7 | Crème eau/huile | 0.4 | S/N | A/N |
| 8 | Shampoing | 0.5 | S/N | S/N |
| 9 | Désodorisant (spray) | 1.3 | S/N | S/N |
| 10 | Spray laque | 0.2 | S/N | S/N |
| 11 | Savon (suif + huile de coco) | 0.5 | S/N | S/LC |
| 12 | Talc | 0.5 | S/N | A/N |

(suite)

| | Article | Conc. (% en poids) | Odeur/aspect [25°C] | Odeur/aspect [40°C] |
|---|---|---|---|---|
| 13 | Détergent en poudre | 0.2 | S/N | S/N |
| 14 | Antiperspirant roll-on | 0.5 | S/N | A/LC |
| 15 | Poudre à récurer chlorée | 0.2 | S/N | S/N |

Clé des abréviations :
S = stable
N = normal
A = acceptable
LC = légère coloration

Comme il ressort de ce tableau, le composé de l'invention convient parfaitement au parfumage d'articles de consommation variés. Il a été constaté qu'il couvrait efficacement l'odeur de la base, là où il y avait lieu, impartissant à ces produits une odeur à la fois fleurie-rosée, fruitée et balsamique-myrrhe, violette très élégante et agréable.

Exemple 16

Parfumage de textiles

On a ajouté à une base non parfumée d'adoucissant textile du commerce 0,1% en poids de (E)-1-(2,2,c-3,t-6-tétraméthyl-r-1-cyclohexyl)-2-pentén-1-one pour obtenir un adoucissant parfumé, à l'aide duquel on a ensuite traité un lot standard de textiles lors d'un cycle de lavage dans une machine à laver le linge.

Les textiles ont ensuite été évalués par un panel de 4 experts parfumeurs, à la sortie de la machine et après le séchage. De leurs avis, les textiles développaient une forte et agréable odeur de type iris, violette, mariée à un côté fruité rappelant l'odeur des damascones.

Ce lot de textiles a également été comparé à l'aveugle avec trois autres lots standard de textiles A, B et C traités de façon similaire mais en présence d'adoucissants textiles parfumés à l'aide de composés connus dont les odeurs sont du type fleuri, poudré, violette. Ainsi, un des échantillons d'adoucissant textile contenait 0,1% en poids d'$\alpha$-ionone (origine : Firmenich SA; utilisée pour traiter le lot A), le deuxième contenait 0,1% en poids de (E)-4-(2,2,c-3,t-6-tétraméthyl-r-1-cyclohexyl)-3-butén-2-one (origine : Firmenich SA ; voir EP 449034 ; utilisée dans le traitement du lot B) et le troisième contenait 0,1% en poids d'$\alpha$-irone (origine : Firmenich SA ; utilisée pour le traitement du lot C).

De l'avis des parfumeurs, l'odeur du lot de textiles traité avec l'adoucissant contenant le composé de l'invention se distinguait de l'odeur des lots A, B et C par son joli caractère fruité, unique et tout à fait surprenant lorsque, comme c'est le cas présent, il se trouve combiné aux notes violette et poudrée typiques des produits dont la structure est soi-disant "de type ironé".

Lorsqu'on comparait justement ce caractère iris-violette des quatre lots de textiles, il a été trouvé que la connotation de l'odeur des textiles parfumés avec le composé de l'invention était plus proche de celle du lot de textiles B, de par son caractère rappelant la myrrhe, que l'on ne retrouve pas dans les lots A et C.

**Revendications**

**1.** Composé de formule

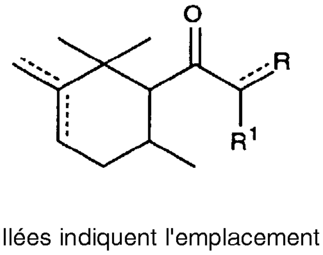

(I)

dans laquelle les lignes pointillées indiquent l'emplacement d'une liaison simple ou double, le symbole $R^1$ représente un atome d'hydrogène ou un radical méthyle et le symbole R représente l'hydrogène ou un radical alkyle, alcényle ou alkylidène contenant de 1 à 4 atomes de carbone, linéaire ou ramifié.

**2.** Composé selon la revendication 1, sous forme d'un isomère racémique ou optiquement actif de formule

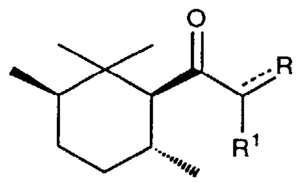

(Ia)

dans laquelle les symboles R et $R^1$ ont le sens indiqué à la formule (I).

**3.** Composé choisi dans le groupe constitué par :

a. (E)-1-(2,2,c-3,t-6-tétraméthyl-r-1-cyclohexyl)-2-pentén-1-one ;
b. cis,(E)-1-(2,2,6-triméthyl-3-méthylène-1-cyclohexyl)-2-butén-1-one ;
c. 1-(2,2,c-3,t-6-tétraméthyl-r-1-cyclohexyl)-1-propanone ; et
d. cis-1-(2,2,3,6-tétraméthyl-3-cyclohexén-1-yl)-1-propanone.

**4.** Utilisation d'un composé selon l'une des revendications 1 à 3 à titre d'ingrédient parfumant.

**5.** Composition parfumante ou article parfumé contenant en tant qu'ingrédient actif un composé selon l'une des revendications 1 à 3.

**6.** Article parfumé selon la revendication 5, sous forme d'un parfum ou d'une eau de toilette, d'un savon, d'un gel de douche ou bain, d'un shampoing ou autre produit d'hygiène capillaire, d'une préparation cosmétique, d'un désodorisant corporel ou d'air ambiant, d'un détergent ou d'un adoucissant textile, ou d'un produit d'entretien.

**7.** Procédé pour la préparation d'un composé de formule (I) telle que définie à la revendication 1, caractérisé en ce qu'il comprend la réaction, avec un agent oxydant, d'un alcool de formule

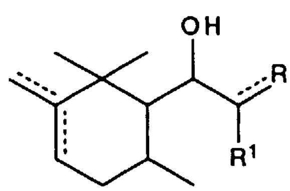

(II)

dans laquelle les lignes pointillées et les symboles $R^1$ et R ont le sens indiqué à la formule (I) et, si nécessaire, l'isomérisation de la cétone ainsi obtenue.

**8.** Procédé selon la revendication 7, caractérisé en ce que ledit alcool se présente sous forme d'un isomère racémique ou optiquement actif de formule

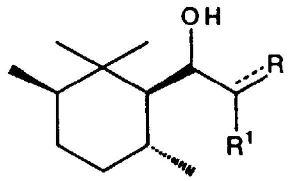

(IIa)

dans laquelle la ligne pointillée et les symboles R et $R^1$ ont le sens indiqué à la formule (I).

**9.** Alcool de formule

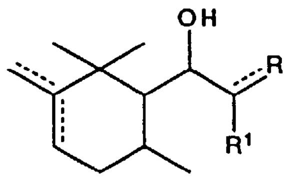

(II)

dans laquelle les lignes pointillées et les symboles R et $R^1$ ont le sens indiqué à la formule (I).

**10.** Alcool selon la revendication 9, sous forme d'un isomère racémique ou optiquement actif de formule

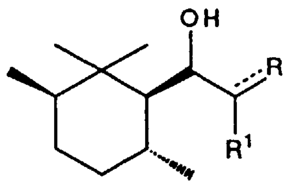

(IIa)

dans laquelle la ligne pointillée et les symboles R et $R^1$ ont le sens indiqué à la revendication 9.

**Patentansprüche**

**1.** Verbindung der Formel

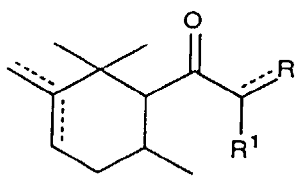

(I)

worin die punktierten Linien die Stellung einer Einfach- oder Doppelbindung anzeigen, das Symbol $R^1$ für ein Wasserstoffatom oder einen Methylrest steht und das Symbol R ein Wasserstoffatom oder einen geradekettigen oder verzweigten Alkyl-, Alkenyl- oder Alkylidenrest mit 1 bis 4 Kohlenstoffatomen bedeutet.

**2.** Verbindung gemäss Patentanspruch 1, in Form eines racemischen oder optisch aktiven Isomeren der Formel

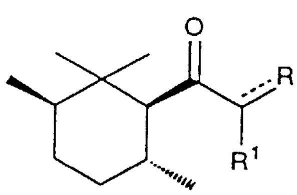

(Ia)

worin die Symbole R und $R^1$ die zu der Formel (I) angegebene Bedeutung besitzen.

**3.** Verbindung ausgewählt aus der Gruppe, bestehend aus:

a. (E)-1-(2,2,c-3,t-6-Tetramethyl-r-1-cyclohexyl)-2-penten-1-on;
b. cis, (E)-1-(2,2,6-Trimethyl-3-methylen-1-cyclohexyl)-2-buten-1-on;
c. 1-(2,2,c-3,t-6-Tetramethyl-r-1-cyclohexyl)-1-propanon; und
d. cis-1-(2,2,3,6-Tetramethyl-3-cyclohexen-1-yl)-1-propanon.

**4.** Verwendung einer Verbindung gemäss einem der Patentansprüche 1 bis 3 als Riechstoffbestandteil.

**5.** Riechstoffkomposition oder parfümierter Artikel enthaltend als aktiven Bestandteil eine Verbindung gemäss einem der Patentansprüche 1 bis 3.

**6.** Parfümierter Artikel gemäss Patentanspruch 5 in Form eines Parfüms oder eines Eau de Toilette, einer Seife, eines Dusch- oder Badegels, eines Shampoos oder eines anderen Haarpflegeproduktes, einer kosmetischen Zubereitung, eines Körperdesodorans oder eines Raumluftverbesserers, eines Detergens oder Textilweichspülers oder eines Universalhaushaltreinigers.

**7.** Verfahren zur Herstellung einer Verbindung der Formel (I), die im Patentanspruch 1 definiert wird, dadurch gekennzeichnet, dass es die Reaktion eines Alkohols der Formel

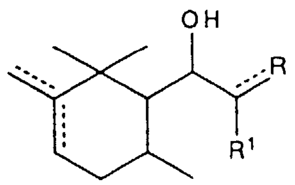

(II)

worin die punktierten Linien und die Symbole $R^1$ und R die zu der Formel (I) angegebene Bedeutung besitzen, mit einem Oxydationsmittel und-falls notwendig-die Isomerisierung des so erhaltenen Ketons, umfasst.

**8.** Verfahren gemäss Patentanspruch 7, dadurch gekennzeichnet, dass sich der Alkohol in Form eines racemischen oder optisch aktiven Isomeren der Formel

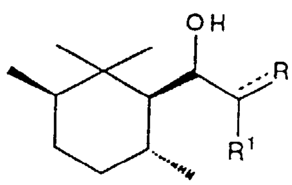

(IIa)

befindet, worin die punktierte Linie und die Symbole R und $R^1$ die zu der Formel (I) angegebene Bedeutung besitzen.

**9.** Alkohol der Formel

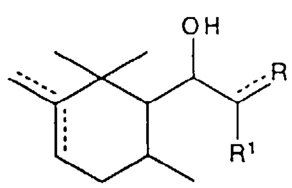

(II)

worin die punktierten Linien und die Symbole R und $R^1$ die zu der Formel (I) angegebene Bedeutung besitzen.

**10.** Alkohol gemäss dem Patentanspruch 9 in Form eines racemischen oder optisch aktiven Isomeren der Formel

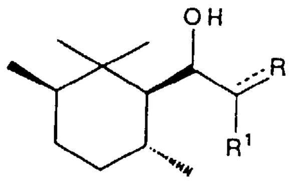

(IIa)

worin die punktierte Linie und die Symbole R und $R^1$ die im Patentanspruch 9 angegebene Bedeutung besitzen.

**Claims**

1. Compound of formula

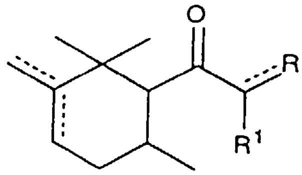

(I)

wherein the dotted lines indicate the location of a single or double bond, symbol $R^1$ represents a hydrogen atom or a methyl group and symbol R represents hydrogen or an alkyl, alkenyl or alkylidene group containing from 1 to 4 carbon atoms, linear or branched.

2. Compound according to claim 1, in the form of a racemic or optically active isomer of formula

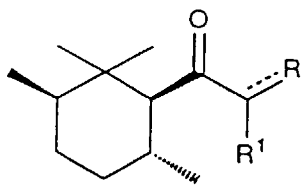

(Ia)

wherein symbols R and $R^1$ have the meaning indicated in formula (I).

3. Compound selected from the group consisting of:

   a. (E)-1-(2,2,c-3,t-6-tetramethyl-r-1-cyclohexyl)-2-penten-1-one ;
   b. cis,(E)-1-(2,2,6-trimethyl-3-methylene)-1-cyclohexyl)-2-buten-1-one ;
   c. 1-(2,2,c-3,t-6-tetramethyl-r-1-cyclohexyl)-1-propanone ; and
   d. cis-1-(2,2,3,6-tetramethyl-3-cyclohexen-1-yl)-1-propanone.

4. Use of a compound according to one of claims 1 to 3 as a perfuming ingredient.

5. Perfuming composition or perfumed article containing as active ingredient a compound according to any one of claims 1 to 3.

6. Perfumed article according to claim 5, in the form of a perfume or a cologne, a soap, a bath or shower gel, a shampoo or other hair-care product, a cosmetic preparation, a body deodorant or an air-freshener, a detergent or a fabric softener, or a household product.

7. Process for the preparation of a compound of formula (I) as defined in claim 1, characterized in that it comprises

the reaction, with an oxidising agent, of an alcohol of formula

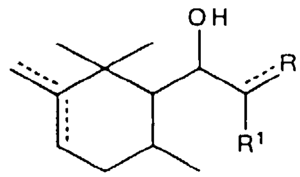

(II)

wherein the dotted lines and symbols $R^1$ and R have the meaning indicated in formula (I) and, if necessary, the isomerisation of the ketone thus obtained.

**8.** Process according to claim 7, characterized in that said alcohol is in the form of a racemic or optically active isomer of formula

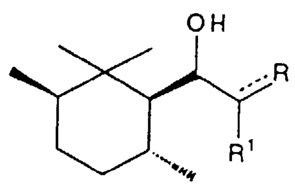

(IIa)

wherein the dotted line and symbols R and $R^1$ have the meaning indicated in formula (I).

**9.** Alcohol of formula

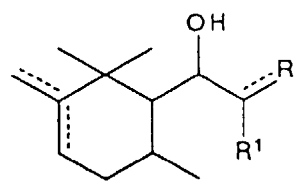

(II)

wherein the dotted lines and symbols R and $R^1$ have the meaning indicated in formula (I).

**10.** Alcohol according to claim 9, in the form of a racemic or optically active isomer of formula

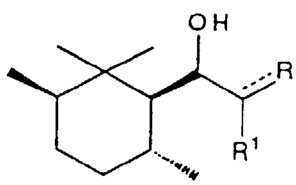

(IIa)

wherein the dotted line and symbols R and $R^1$ have the meaning indicated in claim 9.